# EUROPÄISCHE PATENTSCHRIFT

(11) **EP 0 546 298 B1**
(45) Veröffentlichungstag und Bekanntmachung des Hinweises auf die Patenterteilung: **02.08.2000**
(21) Anmeldenummer: 92118359.6
(22) Anmeldetag: 28.10.1992
(51) Int. Cl.: C07D 239/26, C09K 19/34, C07D 239/34, C07D 213/30, G02F 1/13

(54) **Fettsäureester, welche einen Pyrimidinring enthalten als Komponenten flüssigkristalliner Gemische**
Fatty acids esters containing a pyrimidine ring as component liquid crystalline mixtures
Esters d'acides gras contenant un cycle pyrimidine, comme constituant de mélanges de cristaux liquides

(30) Priorität: 08.11.1991 CH 327391; 18.06.1992 CH 192792
(43) Veröffentlichungstag der Anmeldung: 16.06.1993
(60) Teilanmeldung: 00100640.2
(73) Patentinhaber: Rolic AG, 6301 Zug (CH)
(72) Erfinder: Kelly, Stephen, CH-4313 Möhlin (CH)
(74) Vertreter: Scott, Fiona Penelope Elaine

(56) Entgegenhaltungen:
- EP-A- 0 260 077
- EP-A- 0 289 270
- EP-A- 0 306 195
- EP-A- 0 313 338
- EP-A- 0 319 167
- EP-A- 0 347 941
- EP-A- 0 356 672
- EP-A- 0 401 522
- EP-A- 0 427 166
- DD-A- 257 638
- DE-A- 3 939 982
- CHEMICAL ABSTRACTS, Band 116, Nr. 16, 20. April 1992, Seite 752, Zusammenfassung Nr. 162680n, Columbus, Ohio, US; & JP-A-03 240 755 (NIPPON MINING CO., LTD) 28-10-1991
- MOL. CRYST. AND LIQUID CRYST., Band 208, November 1991, Seiten 9-19, Gordon and Breach Science Publishers S.A., US; G. HEPPKE et al.: "The Sm phase: evidence for a new type of tilted smectic phase"
- MOL. CRYST. AND LIQUID CRYST., Band 199, Mai 1991, Seiten 129-140, Gordon and Breach Science Publishers S.A., US; N. SHIRATORI et al.: "New ferroelectric liquid crystals having 2-fluoro-2-methyl alkanoyloxy group"
- ZEITSCHRIFT FÜR NATURFORSCHUNG B, Band 44, Nr. 9, September 1989, Seiten 1127-1131; J. BÖMELBURG et al.: "Synthese und ferroelektrische Eigenschaften chiraler Ester von 2,5-Diphenylpyrimidinen mit alpha-Fluocarbonsäuren"
- CHEMICAL ABSTRACTS, Band 113, Nr, 22, 26. November 1990, Seite 692, Zusammenfassung Nr. 201537f, Columbus, Ohio, US; & JP-A-02 142 751 (ADEKA ARGUS CHEMICAL CO., LTD) 31-05-1990
- CHEMICAL ABSTRACTS, Band 111, Nr. 22, 27. November 1989, Seite 741, Zusammenfassung Nr. 205651v, Columbus, Ohio, US; & JP-A-01 106 870 (CHISSO CORP.) 24-04-1989
- CHEMICAL ABSTRACTS, Band 110, Nr. 14, 3. April 1989, Seite 761, Zusammenfassung Nr. 125654n, Columbus, Ohio, US; & JP-A-63 230 674 (TOSHIBA CORP.) 27-09-1988
- FERROELECTRICS, Band 85, 1988, Seiten 235-254, US; S. MATSUMOTO et al.: "Multiplexed ferroelectric liquid crystal display"
- CHEMICAL ABSTRACTS, Band 115, Nr. 22, 2. Dezember 1991, Seite 839, Zusammenfassung Nr. 244241p, Columbus, Ohio, US; & JP-A-3 095 164 (NIPPON MINING CO., LTD) 19-04-1991
- CHEMICAL ABSTRACTS, Band 110, Nr. 13, 27. März 1989, Seite 762, Zusammenfassung Nr. 125661, Columbus, Ohio, US; & JP-A-63 253 074 (ADEKA ARGUS CHEMICAL CO., LTD)
- LIQUID CRYSTALS, Band 2, Nr. 4, 1987, Seiten 411-422; V. KRONE: "Liquid-crystalline monomers, dimers and side group polymers contaning phenylpyrimidine mesogens"
- CHEMICAL ABSTRACTS, Band 110, Nr. 24, 12. Juni 1989, Seite 722, Zusammenfassung Nr. 223266g, Columbus, Ohio, US; & JP-A-63 280 064 (SEIKO EPSON CORP.) 17-11-1988
- CHEMICAL ABSTRACTS, Band 117, Nr. 16. 19. Oktober 1992, Seite 708, Zusammenfassung Nr. 161126v, Columbus, Ohio, US; & JP-A-04 134 072 (NIPPON MINING CO., LTD) 07-05-1992
- CHEMICAL ABSTRACTS, Band 116, Nr. 16, 20. April 1992, Seite 752, Zusammenfassung Nr. 162678t, Columbus, Ohio, US; & JP-A-03 263 483 (TOSHIBA CORP.) 22-11-1991
- CHEMICAL ABSTRACTS, Band 117, Nr. 12, 21. September 1992, Seite 695, Zusammenfassung Nr. 121719j, Columbus, Ohio, US; & JP-A-4 029 975 (NIPPON MINING CO., LTD) 31-01-1992

## Beschreibung

Die vorliegende Erfindung betrifft aromatische Ester, flüssigkristalline Gemische, die solche Verbindungen enthalten sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), SSF-Zellen (surface stabilized ferroelectric), DHF-Zellen (deformed helix ferroelectric) oder SBF-Zellen (short-pitch bistable ferroelectric).

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine breite smektische Mesophase.

Da Flüssigkristalle in der Regel als Mischungen mehrerer Komponenten zur Anwendung gelangen, ist es wichtig, dass die Komponenten untereinander gut mischbar sind.

Die vorliegende Erfindung stellt nun Verbindungen, die in hervorragender Weise für solche Flüssigkristall-Mischungen geeignet sind zur Verfügung. Gegenstand der vorliegenden Erfindung sind Verbindungen der allgemeinen Formel 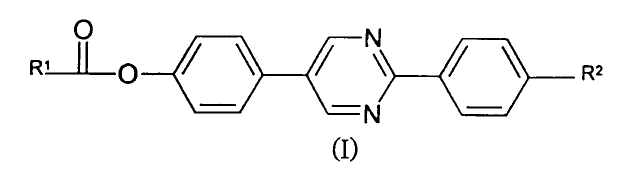 worin
- R¹: unverzweigtes Alkyl oder Alkenyl mit 1 bis 12, bzw. 2 bis 12 Kohlenstoffatomen, in welchen eine Methylengruppe durch -0- ersetzt sein kann, bedeutet; und
- R²: unsubstituiertes oder einfach oder mehrfach mit Halogen substituiertes Alkyl oder Alkenyl mit 1 bis 12, bzw. 2 bis 12 kohlenstoffatomen, in welchen eine oder mehrere Methylengruppen durch -0-, -COO- oder -OOC- ersetzt sein können, bedeutet.

Ähnliche Verbindungen wurden in den Anmeldungen EP-A-0 356 672 und EP-A-0 347 941 schon beschrieben.

Es wurde überraschenderweise gefunden, dass die erfindungsgemässen Verbindungen für ferroelektrische Anwendungen ausgesprochen günstige Mesophasen aufweisen.

Durch Beimischen der erfindungegemässen Verbindungen der allgemeinen Formel I wird in Flüssigkristall-Mischungen oft eine bedeutende Erhöhung des S_{C}-S_{A}, S_{C}-N, S_{A}-I oder N-I Phasenüberganges bewirkt und der Schmelzpunkt signifikant abgesenkt, was zu einer breiten smektischen Mesophase führt. Die erfindungsgemässen Verbindungen weisen breite smektische Mesophasen mit erstaunlich niedriger Viskosität auf. Dadurch können durch Zugabe solcher Verbindungen die Schaltzeiten von Grundmischungen erheblich gesenkt werden. Die erfindungsgemässen zweikernigen Verbindungen erweisen sich trotz ihrer nematischen Phase als ausgezeichnete Komponenten für S_{C}-Mischungen. Sie verschmälern die S_{A}-Phase und induzieren eine nematische Phase, ohne den So-Phasen-Uebergang zu senken. Auf diese Weise ergeben sich Phasen, die sich optimal ausrichten lassen, und die sich so hervorragend für SSF Anwendungen eignen.

Der Ausdruck "unverzweigtes Alkyl oder Alkenyl mit 1 bis 12, bzw. 2 bis 12 Kohlenstoffatomen, in welchen eine Methylengruppe durch -O- ersetzt sein kann" bedeutet im Rahmen der vorliegenden Erfindung Gruppen wie Alkyl, Alkoxy, Alkenyl, Alkenyloxy, Alkoxyalkyl, Alkenyloxyalkyl, Alkoxyalkenyl mit 1 bis 12, bzw. 2 bis 12 Kohlenstoffatomen. Solche Gruppen sind beispielsweise Methyl, Aethyl, Propyl, Butyl, Pentyl, Hexyl, Methoxy, Aethoxy, Propyloxy, Butyloxy, Pentyloxy, Hexyloxy, Methoxypropyl, Methoxybutyl, Methoxypentyl, Aethoxypropyl, Aethoxybutyl, Propyloxypropyl, 1E-Propenyl, 1E-Butenyl, 1E-Pentenyl, 1E-Hexenyl, 1E-Heptenyl, 1E-Octenyl, 1E-Nonenyl, 1E-Decenyl, 1E-Undecenyl und dergleichen.

Der Ausdruck "unsubstituiertes oder einfach oder mehrfach mit Halogen substituiertes Alkyl oder Alkenyl mit 1 bis 12, bzw. 2 bis 12 Kohlenstoffatomen, in welchen eine oder mehrere Methylengruppen durch -O- und/oder -COO- oder -OOC- ersetzt sein können" bedeutet im Rahmen der vorliegenden Erfindung u.a. Gruppen wie vorhergehend definiert. Zusätzlich umfasst dieser Ausdruck Alkenylgruppen wie 2Z-Butenyl, 2Z-Pentenyl, 2Z-Hexenyl, 3-Butenyl, 3E-Pentenyl, 3E-Hexenyl, 4-Pentenyl, 4Z-Hexenyl, 5-Hexenyl, Allyloxy, 2E-Butenyloxy, 3-Butenyloxy, 2E-Pentenyloxy, 3Z-Pentenyloxy, 4-Pentenyloxy, 2E-Hexenyloxy, 3Z-Hexenyloxy, 4E-Hexenyloxy, 2E-Heptenyloxy, 2E-Octenyloxy, 2E-Nonenyloxy, 2E-Decenyloxy, 2E-Undecenyloxy, 2E-Dodecenyloxy. Allyloxypropyl, 3-Butenyloxypropyl, und auch verzweigte oder vorzugsweise unverzweigte Gruppen, welche in der Kette anstelle eines Sauerstoffatomes, oder auch zusätzlich hierzu, eine Carboxylgruppe enthalten. Solche Gruppen sind beispielsweise Alkoxycarbonyl, Alkanoyloxy, Alkoxycarbonylalkyl oder Alkanoyloxyalkyl, Alkenoyloxy, Alkenyloxycarbonyl und dergleichen. Diese Gruppen können einfach oder mehrfach mit Halogen, insbesondere mit Fluor substituiert sein. Solche Gruppen sind beispielsweise Acetoxy, Propanoyloxy, Butanoyloxy, Pentanoyloxy, Hexanoyloxy, Aethoxycarbonyl, Propyloxycarbonyl, Butyloxycarbonyl, Pentyloxycarbonyl, Hexyloxycarbonyl, 2E-Butenoyloxy, Pentenoyloxy, Hexenoyloxy, 2E-Butenoyloxy, 2E-Pentenoyloxy, 2E-Hexenoyloxy, 2E-Heptenoyloxy, 2E-Octenoyloxy, 2E-Nonenoyloxy, 2E-Decenoyloxy, 2E-Undecenoyloxy, 2E-Dodecenoyloxy, Allyloxycarbonyl, 2-Butenyloxycarbonyl, 3-Butenyloxycarbonyl, 2-Pentenyloxycarbonyl, 3-Pentenyloxycarbonyl, 4-Pentenyloxycarbonyl und dergleichen. Bevorzugt sind hier jedoch Gruppen mit 1 bis 7, bzw. 2 bis 7 Kohlenstoffatomen.

Der Ausdruck "Halogen" umfasst im Rahmen der vorliegenden Erfindung Fluor, Chlor, Brom und Jod, insbesondere jedoch Fluor.

Die erfindungegemässen Verbindungen der allgemeinen Formel I sind synthetisch leicht zugänglich und können in an sich bekannter Weise aus Phenolen bzw. 5-Hydroxypyrimidinen und einer Säure hergestellt werden. Die Veresterung kann beispielsweise in Gegenwart von N,N'-Dicyclohexylcarbodiimid und 4-(Dimethylamino)pyridin in Dichlormethan oder einem anderen geeigneten Lösungsmittel wie z.B. Chloroform erfolgen. Die Ausgangsmaterialien sind bekannt und z.T. im Handel erhältlich, oder sie können wie z.B. in den nachfolgenden Schemata 1 bis 3 angegeben, hergestellt werden.

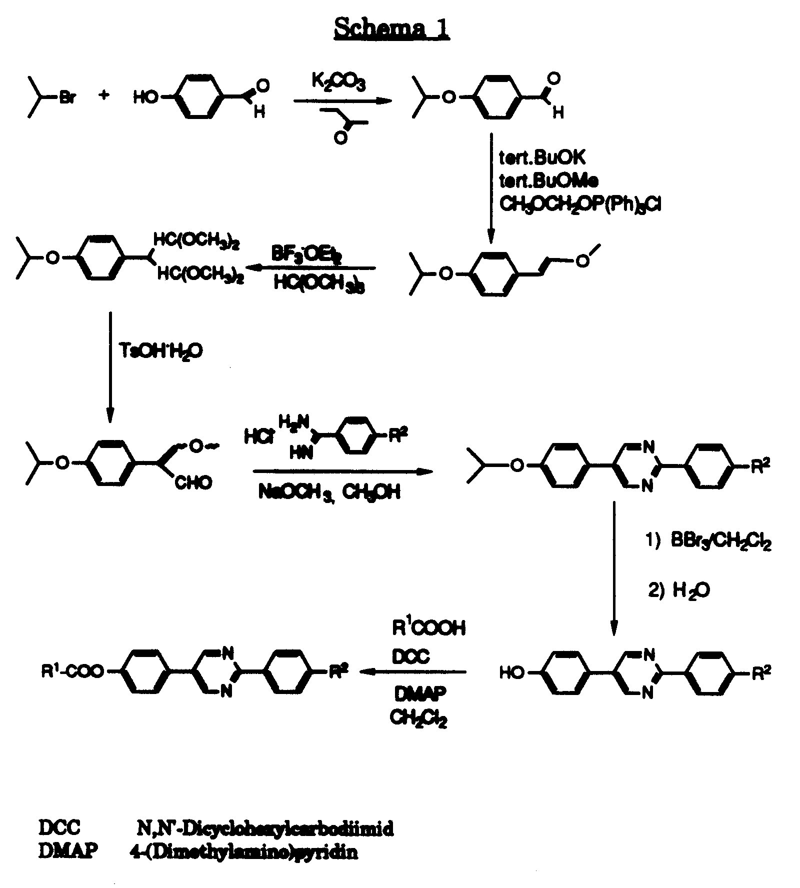

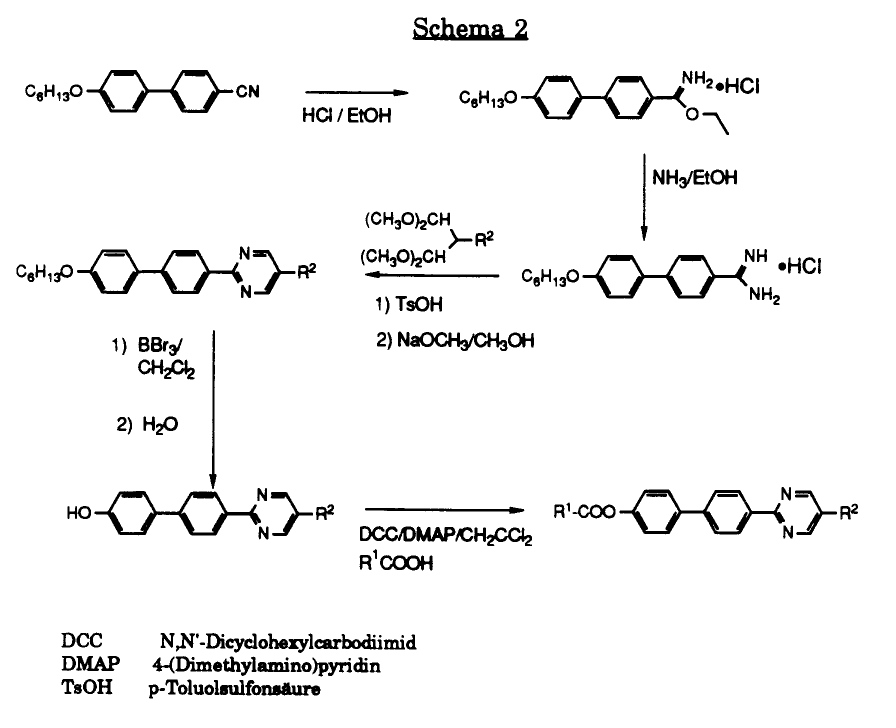

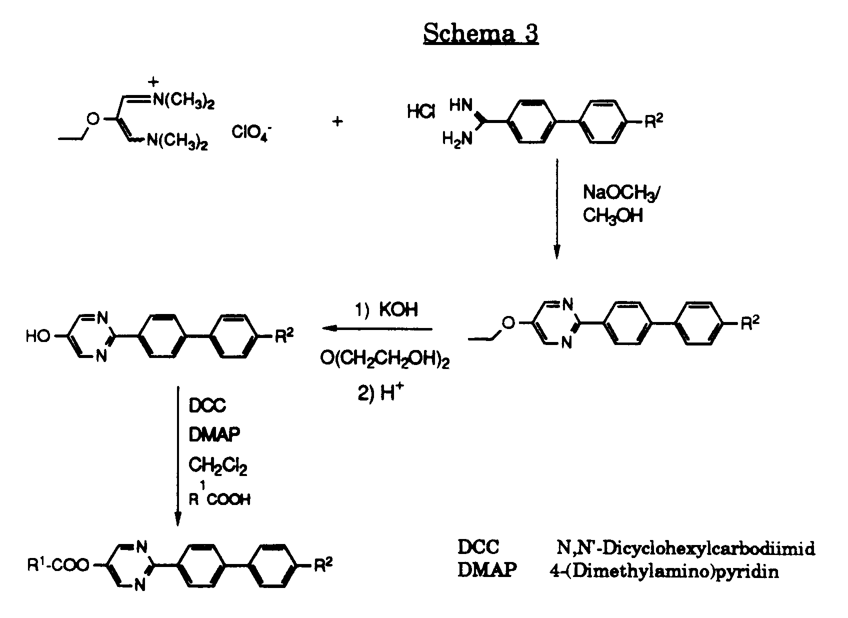

Die Verbindungen der Formel I können in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere, bekannte flüssigkristalline Verbindungen sein. Es muss jedoch jeweils mindestens eine chirale Komponente im Gemisch enthalten sein. Sofern nicht bereits eine der verwendeten Komponenten chiral ist, muss demnach ein chiraler Dotierstoff zugesetzt werden.

Derartige Flüssigkristallkomponenten sind vorzugsweise achirale Verbindungen der Formeln 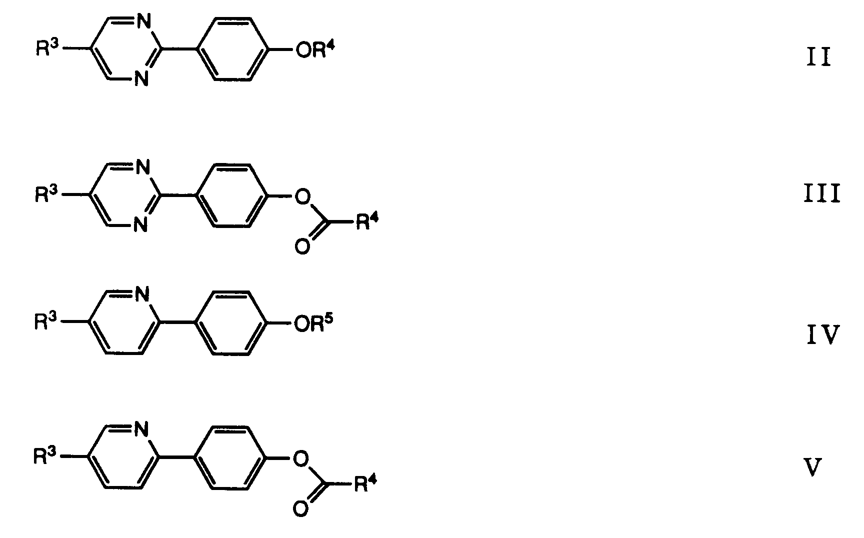 bzw. chirale Dotierstoffe der allgemeinen Formeln 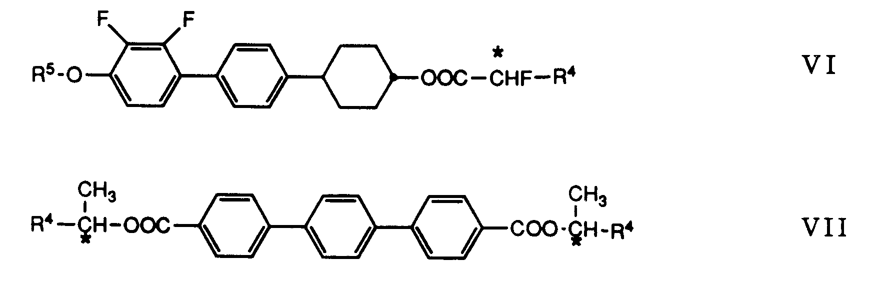 worin R³ Alkyl oder Alkoxy darstellt, R⁴ Alkyl bedeutet, und R⁵ Alkyl oder Alkenyl bedeutet.

Der Ausdruck "Alkyl" im Zusammenhang mit den Verbindungen der Formeln II bis VII umfasst unverzweigte oder verzweigte Alkylgruppen mit 1 bis 12 Kohlenstoffatomen, vorzugsweise unverzweigte Alkylgruppen mit 6 bis 12 Kohlenstoffatomen wie Hexyl, Heptyl, Octyl, Nonyl, Decyl, Undecyl, Dodecyl.

Der Ausdruck "Alkoxy" umfasst Aethergruppen in denen der Alkylrest wie vorhergehend definiert ist.

Der Ausdruck "Alkenyl" umfasst im Zusammenhang mit den Verbindungen der Formeln II-VII Alkenylgruppen mit 2 bis 15 Kohlenstoffatomen, wie 2E-Alkenyl, 3Z-Alkenyl, 4E-Alkenyl und Alkenyl mit endständiger Doppelbindung. Die Ausdrücke "2E-Alkenyl", "3Z-Alkenyl" und 4E-Alkenyl" umfassen vorzugsweise unverzweigte Alkenylgruppen mit 3 bis 9, 4 bis 9 bzw. 5 bis 9 Kohlenstoffatomen, in welchen die Doppelbindung in 2, 3 bzw. 4 Stellung steht, wobei E und Z die Konfiguration der Doppelbindung bezeichnen. Solche Gruppen sind beispielsweise 2E-Butenyl, 2E-Pentenyl, 2E-Hexenyl, 2E-Heptenyl, 2E-Octenyl, 2E-Nonenyl, 3-Butenyl, 3Z-Pentenyl, 3Z-Hexenyl, 3Z-Heptenyl, 3Z-Octenyl, 3Z-Nonenyl, 4-Pentenyl, 4E-Hexenyl, 4E-Heptenyl, 4E-Octenyl, 4E-Nonenyl, 5-Hexenyl, 6-Heptenyl, 7-Octenyl, 8-Nonenyl und dergleichen.

Aufgrund der guten Löslichkeit der Verbindungen der Formel I und aufgrund ihrer guten Mischbarkeit untereinander kann der Anteil an Verbindungen der Formel I in den erfindungsgemässen Gemischen relativ hoch sein und bis etwa 85 Gew.-% betragen. Bevorzugt wird im allgemeinen ein Anteil von etwa 1-50, insbesondere 5-30 Gew.-% an Verbindungen der Formel I.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, Ch eine chiral nematische, S eine smektische und I die isotrope Phase.

### Beispiel 1

Zu einer Lösung von 1,0 g 4-(2-[4-Pentylphenyl]-5-pyrimidinyl)phenol, 0,4 g Caprinsäure und 0,05 g 4-(Dimethylamino)pyridin in 30 ml Dichlormethan wird unter Rühren innert 5 Minuten 0,8 g N,N'-Dicydohexylcarbodiimid gegeben. Das Reaktionsgemisch wird über Nacht weiter gerührt, dann filtriert, das Filtrat mit gesättigter Natriumbicarbonat-Lösung und mit Wasser gewaschen und eingeengt. Chromatographie des Rückstandes an Kieselgel mit Hexan/Aethylacetet (Vol. 4:1) und zweimaliges Umkristallisieren der gemäss Dünnschichtchromatographie reinen Fraktionen ergibt 1,0 g 4-(2-[4-Pentylphenyl]-5-pyrimidinyl)phenylhexanoat; Smp. (C-S_{C}) 124°C, Phasenübergang (S_{C}-S_{A}) 185°C, Klp. (S_{A}-I) 224°C.

Das als Ausgangsmaterial verwendete 4-(2-[4-Pentylphenyl]-5-pyrimidinyl)phenol wird wie folgt hergestellt:
a) Eine Lösung von 30 g 4-Hydroxybenzaldehyd und 46 g Isopropylbromid in 300 ml N,N-Dimethylformamid wird mit 104 g fein pulverisiertem Kaliumcarbonat versetzt und das Gemisch über Nacht bei 58°C gerührt. Die Suspension wird genutscht und das Filtrat im Vakuum eingeengt. Eine Lösung des Rückstands in 100 ml Diäthyläther wird zweimal mit je 100 ml Wasser gewaschen, über Natriumsulfat getrocknet und eingeengt. Chromatographie des Rohproduktes an Kieselgel mit Hexan/Aethylacetat (Vol. 9:1) ergibt 40 g 4-(Isopropyloxy)benzaldehyd.
b) Eine Suspension von 140 g Methoxymethyl-triphenylphosphoniumchlorid in 400 ml tert.-Butyl-methyläther wird unter Stickstoffbegasung bei 0°C innert 5 Minuten mit 45g Kalium-tert.-butylat versetzt und noch 10 Minuten gerührt. Sodann wird bei 0°C innert 30 Minuten eine Lösung von 41 g 4-(Isopropyloxy)-benzaldehyd in 100 ml tert.-Butyl-methyläther zugetropft. Das Reaktionsgemisch wird noch 16 Stunden bei Raumtemperatur gerührt und dann auf 750 ml 0,8N Natriumhydrogencarbonat-Lösung gegossen. Die wässrige Phase wird abgetrennt und mit Diäthyläther nachextrahiert. Die organische Phase wird mit Wasser neutral gewaschen, über Magnesiumsulfat getrocknet und eingeengt. Der Rückstand wird in 800 ml Hexan aufgeschlämmt. Nach Abkühlen auf 0°C wird das Triphenylphosphinoxid abfiltriert und das Filtrat eingeengt. Chromatographie des Rückständes an Kieselgel mit Hexan/Aethylacetat (Vol. 9:1) ergibt 45 g reines 1-Methoxy-2-(4-[isopropoxy]phenyl)äthen.
c) Unter Stickstoffatmosphäre werden bei 0°C zu 273 ml Orthoameisensäuretrimethylester zuerst 10 ml Bortrifluorid-diäthylätherat zugegeben und anschliessend 48 g 1-Methoxy-2-(4-[isopropoxy]phenyl)äthen zugetropft. Das Reaktionsgemisch wird noch 4 Stunden bei 0°C gerührt und dann mit 6 ml Triäthanolamin neutralisiert und eingeengt. Der Rückstand wird in 500 ml Diäthyläther aufgenommen und die Lösung mit 100 ml gesättigter Natriumbicarbonat-Lösung und zweimal mit je 150 ml Wasser gewaschen, über Magnesiumsulfat getrocknet, filtriert und eingeengt. Chromatographie des Rückstandes an Kieselgel mit Hexan/Aethylacetat (Vol. 9:1) und Destillation bei 135-137°C (1 mmHg) ergibt 44 g (4-[Isopropoxy]phenyl)malonaldehyd-tetramethylacetal.
d) Unter Stickstoffatmosphäre wird ein Gemisch aus 20,9 g (4-[Isopropyloxy]phenyl)malonaldehyd-tetramethylacetal 5,0 g p-Toluolsulfonsäuremonohydrat und 1,5 ml Wasser 2 Stunden auf 70-80°C erhitzt, dann mit 0,3 g Natriumbicarbonat versetzt, noch 5 Minuten gerührt und filtriert. Der Filterrückstand wird mit Methanol nachgewaschen und das Filtrat mit dem entstandenen rohen 2-(Methoxymethyliden)-2-(4-[isopropoxy]phenyl)acetaldehyd sofort in der nächsten Stufe eingesetzt.
e) Eine aus 14,7 ml Methanol und 2,5 g Natrium frisch hergestellte Natriummethylat-Lösung wird unter Stickstoffatmosphäre zu einem Gemisch aus 15,0 g p-Pentylbenzamidin-hydrochlorid, 75 ml Methanol und der obigen methanolischen Lösung von 2-(Methoxymethyliden)-2-(4-[isopropyloxy]phenyl)acetaldehyd zugetropft. Das Reaktionsgemisch wird über Nacht gerührt und anschliessend mit konzentrierter Salzsäure auf pH 3-4 gestellt. Das ausgefallene 5-(4-Isopropyloxyphenyl)-2-(4-pentylphenyl)pyrimidin wird abfiltriert, mit Wasser und mit Methanol gewaschen und im Vakuum getrocknet. Chromatographie des Rückstandes an Kieselgel mit Dicblormethan/Hexan (Vol. 1:1) ergibt 6,6 g reines Produkt.
f) Eine Lösung von 5,5 g Bortribromid in 50 ml absolutem Dichlormethan wird bei 0°C tropfenweise mit einer Lösung von 6,5 g 5-(4-Isopropyloxyphenyl)-2-(4-pentylphenyl)pyrimidin in 50 ml absolutem Dichlormethan versetzt. Das Gemisch wird 24 Stunden gerührt und dann auf Eiswasser gegossen. Die organische Phase wird abgetrennt und die wässrige Phase dreimal mit je 50 ml Dichlormethan nachextrahiert. Die vereinigten organischen Phasen wird mit 50 ml 2N Natriumcarbonat-Lösung und mehrmals mit Wasser gewaschen, über Magnesiumsulfat getrocknet und eingeengt. Chromatographie des Rückstandes an Kieselgel mit Dichlormethan/Methanol (Vol. 99:1) und Umkristallisation aus Hexan ergibt 5,2 g 4-(2-[4-Pentylphenyl]-5-pyrimidinyl)phenol mit Smp. 152-153°C.

In analoger Weise können folgende Verbindungen hergestellt werden:
4-(2-[4-Propylphenyl]-5-pyrimidinyl)phenyl-acetat;
4-(2-[4-Propylphenyl]-5-pyrimidinyl)phenyl-propionat;
4-(2-[4-Propylphenyl]-5-pyrimidinyl)phenyl-butanoat;
4-(2-[4-Propylphenyl]-5-pyrimidinyl)phenyl-pentanoat;
4-(2-[4-Propylphenyl]-5-pyrimidinyl)phenyl-hexanoat;
4-(2-[4-Propylphenyl]-5-pyrimidinyl)phenyl-heptanoat;
4-(2-[4-Pentylphenyl]-5-pyrimidinyl)phenyl-acetat. Smp. (C-S_{C}) 169°C, S_{C}-S_{A} 137°C, S_{A}-N 179°C, Klp. (N-I) 222°C;
4-(2-[4-Pentylphenyl]-5-pyrimidinyl)phenyl-propionat, Smp. (C-S_{C}) 150°C, S_{C}-S_{A} 166°C, S_{A}-N 217°C, Klp. (N-I) 230°C;
4-(2-[4-Pentylphenyl]-5-pyrimidinyl)phenyl-butanoat Smp. (C-S_{C}) 143°C, S_{C}-S_{A} 170°C, S_{A}-N 225°C, Klp. (N-I) 229°C;
4-(2-[4-Pentylphenyl]-5-pyrimidinyl)phenyl-pentanoat, Smp. (C-S_{C}) 139°C, S_{C}-S_{A} 184°C, Klp. (S_{A}-I) 224°C;
4-(2-[4-Pentylphenyl]-5-pyrimidinyl)phenyl-hexanoat,Smp. (C-S₃) 124°C, S₃-S_{C} 132°C, S_{C}-S_{A} 184°C, Klp. (S_{A}-I) 224°C;
4-(2-[4-Pentylphenyl]-5-pyrimidinyl)phenyl-heptanoat;
4-(2-[4-Pentylphenyl]-5-pyrimidinyl)phenyl-octanoat Smp. (C-S₃) 110°C, S₃-S_{C} 134°C, S_{C}-S_{A} 197°C, Klp. (S_{A}-I) 217°C;
4-(2-[4-Heptylphenyl]-5-pyrimidinyl)phenyl-acetate;
4-(2-[4-Heptylphenyl]-5-pyrimidinyl)phenyl-propionat;
4-(2-[4-Heptylphenyl]-5-pyrimidinyl)phenyl-butanoat;
4-(2-[4-Heptylphenyl]-5-pyrimidinyl)phenyl-pentanoat;
4-(2-[4-Heptylphenyl]-5-pyrimidinyl)phenyl-hexanoat;
4-(2-[4-Heptylphenyl]-5-pyrimidinyl)phenyl-heptanoat.

### Beispiel 2

Zur Untersuchung der Eigenschaften der Verbindungen der Formel I wird eine Grundmischung hergestellt und jeweils 20% einer Verbindung der Formel I zugemischt. Die Phasenübergangstemperaturen dieser Mischungen werden bestimmt, die Kristallisationstemperatur T_{c} wird dabei aus Leitfähigkeitsdaten bestimmt. Die Schaltzeiten werden bei 25°C (10 Vpp/µ, Zeit vom Start des Pulses bis zum Maximum des Stromes) gemessen. Die Messwerte sind in der Tabelle 1 zusammengefasst.

### Grundmischung

27,6 Gew.-% Bis[(S)-1-methylheptyl]-p-terphenyl 4,4'-dicarbonsäureester
18,4 Gew.-% 2-[4-(Hexyloxy)phenyl]-5-nonylpyrimidin
18,4 Gew.-% 2-[4-(Nonyloxy)phenyl]-5-nonylpyrimidin
9,2 Gew.-% 2-[4-(Nonyloxy)phenyl]-5-octylpyrimidin
9,2 Gew.-% 2-[4-(Heptyloxy)phenyl]-5-heptylpyrimidin
9,2 Gew.-% 2-[4-(Decyloxy)phenyl]-5-octylpyrimidin
8,0 Gew.-% 4-(Decyloxy)benzoesäure-4-(2-[trans-4-pentylcyclohexyl]-1-äthyl)phenylester.

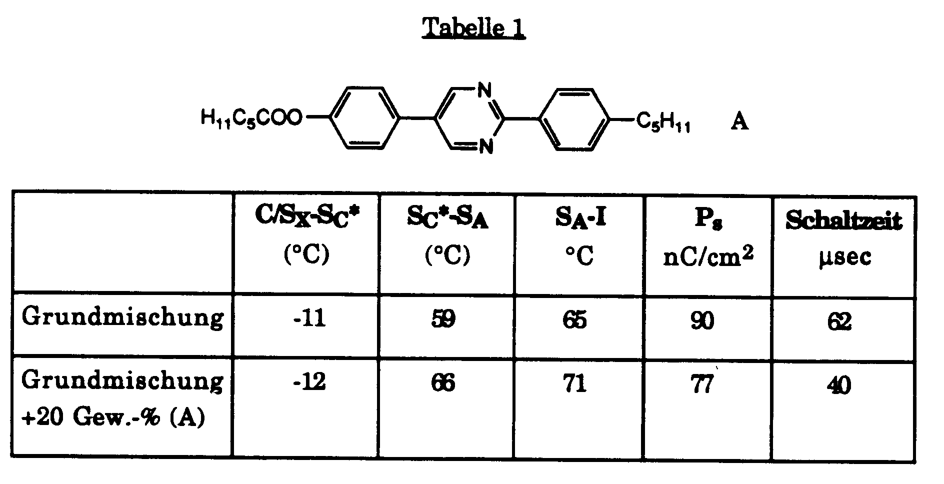

## Patentansprüche

1. Verbindung der allgemeinen Formel (I) 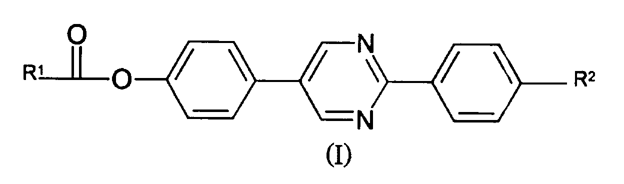 worin
R¹ unverzweigtes Alkyl oder Alkenyl mit 1 bis 12, bzw. 2 bis 12 Kohlenstoffatomen, in welchen eine Methylengruppe durch -0- ersetzt sein kann, bedeutet; und
R² unsubstituiertes oder einfach oder mehrfach mit Halogen substituiertes Alkyl oder Alkenyl mit 1 bis 12, bzw. 2 bis 12 Kohlenstoffatomen, in welchen eine oder mehrere Methylengruppen durch -0-, -COO- oder -OOC- ersetzt sein können, bedeutet.

2. Verbindung gemäss Anspruch 1, dadurch gekennzeichnet, dass R² ein Alkyl mit 1 bis 7 Kohlenstoffatomen oder ein Alkenyl mit 2 bis 7 Kohlenstoffatomen ist.

3. Flüssigkristalline Mischung enthaltend mindestens zwei Verbindungen, wovon mindestens eine Verbindung die allgemeine Formel I hat, 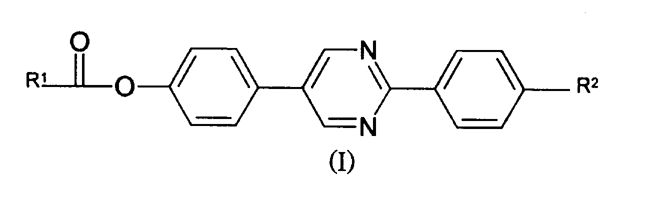 wobei
R¹ unverzweigtes Alkyl oder Alkenyl mit 1 bis 12, bzw. 2 bis 12 Kohlenstoffatomen, in welchen eine Methylengruppe durch -0- ersetzt sein kann, bedeutet; und
R² unsubstituiertes oder einfach oder mehrfach mit Halogen substituiertes Alkyl oder Alkenyl mit 1 bis 12, bzw. 2 bis 12 Kohlenstoffatomen, in welchen eine oder mehrere Methylengruppen durch -0-, -COO- oder -OOC- ersetzt sein können, bedeutet.

4. Flüssigkristalline Mischung nach Anspruch 3, dadurch gekennzeichnet, dass es eine ferroelektrische Mischung ist.

5. Verwendung einer Verbindung gemäss Anspruch 1 oder 2 für elektro-optische Zwecke.

6. Elektrooptische Vorrichtung enthaltend eine Verbindung gemäss Anspruch 1 oder 2 oder eine flüssigkristalline Mischung gemäss Anspruch 3 oder 4.

## Claims

1. Compound of the general formula (I) 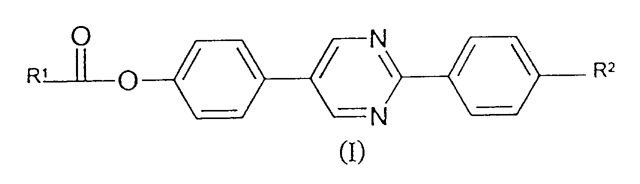 wherein
R¹ signifies unbranched alkyl or alkenyl having from 1 to 12 or, respectively, from 2 to 12 carbon atoms, in which one methylene group can be replaced by -O-; and
R² signifies alkyl or alkenyl having from 1 to 12 or, respectively, from 2 to 12 carbon atoms, which is unsubstituted or mono- or poly-substituted by halogen and in which one or more methylene groups can be replaced by -O-, -COO- or -OOC-.

2. Compound according to claim 1, characterised in that R² is alkyl having from 1 to 7 carbon atoms or alkenyl having from 2 to 7 carbon atoms.

3. Liquid crystalline mixture comprising at least two compounds, of which at least one compound has the general formula I 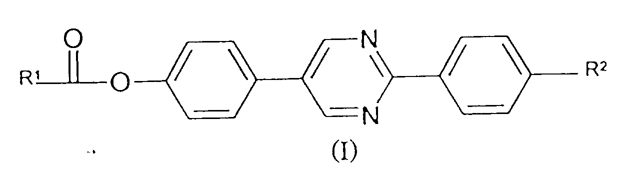 wherein
R¹ signifies unbranched alkyl or alkenyl having from 1 to 12 or, respectively, from 2 to 12 carbon atoms, in which one methylene group can be replaced by -O-; and
R² signifies alkyl or alkenyl having from 1 to 12 or, respectively, from 2 to 12 carbon atoms, which is unsubstituted or mono- or poly-substituted by halogen and in which one or more methylene groups can be replaced by -O-, -COO- or -OOC-.

4. Liquid crystalline mixture according to claim 3, characterised in that it is a ferroelectric mixture.

5. The use of a compound according to claim 1 or 2 for electro-optical purposes.

6. Electro-optical device comprising a compound according to claim 1 or 2 or a liquid crystalline mixture according to claim 3 or 4.

## Revendications

1. Composé de formule générale (I) 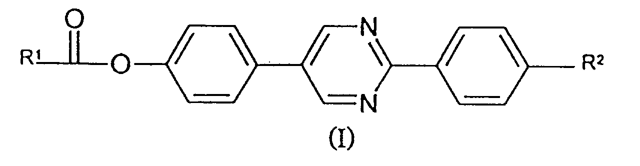 dans laquelle
R¹ représente un groupe alkyle ou alcényle non ramifié ayant de 1 à 12, respectivement de 2 à 12 atomes de carbone, dans lesquels un groupe méthylène peut être remplacé par -O-; et
R² représente un groupe alkyle ou alcényle non substitué ou substitué une ou plusieurs fois par un atome d'halogène, ayant de 1 à 12, respectivement de 2 à 12 atomes de carbone, dans lesquels un ou plusieurs groupes méthylène peuvent être remplacés par -O-, -COO- ou -OOC-.

2. Composé selon la revendication 1, caractérisé en ce que R² est un groupe alkyle ayant de 1 à 7 atomes de carbone ou un groupe alcényle ayant de 2 à 7 atomes de carbone.

3. Composition à cristaux liquides contenant au moins deux composés, parmi lesquels au moins un composé a la formule générale I, 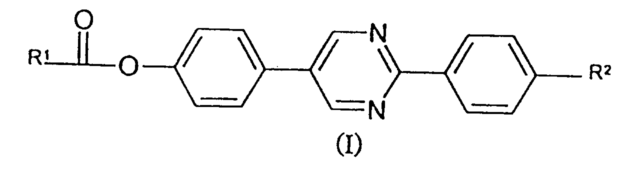 dans laquelle
R¹ représente un groupe alkyle ou alcényle non ramifié ayant de 1 à 12, respectivement de 2 à 12 atomes de carbone, dans lesquels un groupe méthylène peut être remplacé par -O-; et
R² représente un groupe alkyle ou alcényle non substitué ou substitué une ou plusieurs fois par un atome d'halogène, ayant de 1 à 12, respectivement de 2 à 12 atomes de carbone, dans lesquels un ou plusieurs groupes méthylène peuvent être remplacés par -O-, -COO- ou -OOC-.

4. Composition à cristaux liquides selon la revendication 3, caractérisée en ce que qu'elle est une composition ferro-électrique.

5. Utilisation d'un composé selon la revendication 1 ou 2 pour des applications électro-optiques.

6. Dispositif électro-optique contenant un composé selon la revendication 1 ou 2 ou une composition à cristaux liquides selon la revendication 3 ou 4.
